Europäisches Patentamt

European Patent Office

Office européen des brevets

(11) Publication number: 0 170 269
A2

# EUROPEAN PATENT APPLICATION

(21) Application number: 85109601.6

(22) Date of filing: 31.07.85

(51) Int. Cl.⁴: **A 61 K 7/00**
**A 61 K 9/12**

(30) Priority: 02.08.84 JP 163342/84
02.08.84 JP 163343/84
03.08.84 JP 163672/84
03.08.84 JP 163673/84

(43) Date of publication of application:
05.02.86 Bulletin 86/6

(84) Designated Contracting States:
AT CH DE FR GB IT LI NL

(71) Applicant: Kao Corporation
14-10, Nihonbashi Kayabacho 1 chome
Chuo-Ku Tokyo 103(JP)

(72) Inventor: Yamamoto, Hiromi
6-23-18, Nakashizu
Sakura-shi Chiba-ken(JP)

(72) Inventor: Yorozu, Hidenori
423-4, Hiramatsuhonmachi
Utsunomiya-shi Tochigi-ken(JP)

(74) Representative: Wächtershäuser, Günter, Dr.
Tal 29
D-8000 München 2(DE)

(54) Medicated cosmetic compositions.

(57) A medicated cosmetic composition comprising at least one medicament selected from the group consisting of antiseborrheics, bactericides, anti-inflammatory agents and refrigerants by 0.001 to 5 wt % in total further comprises carbon dioxide or a substance capable of emitting carbon dioxide by 60 ppm or more.

Owing to the incorporation of carbon dioxide or a substance capable of emitting carbon dioxide, medical effects of the medicaments are drastically improved compared with the case where they are used singly.

The medical cosmetic composition may be manufactured into lotions, emulsions, hair tonics, shampooes, rinses and the like and is prefarably charged in a pressure-resistant container.

EP 0 170 269 A2

## BACKGROUND OF THE INVENTION

### i)    Field of the Invention:

This invention relates to medicated cosmetic compositions and more particularly, to medicated cosmetic compositions which comprise at least one medicament selected from the group consisting of antiseborrheics, bactericides, antiinflammatory agents and refrigerants, and carbon dioxide or a substance capable of emitting carbon dioxide.

### ii)    Description of the Prior Art:

At present, attempts have been frequently made to add, to cosmetic compositions, medicaments as well as ordinary cosmetic ingredients. For instance, there are used antiseborrheics so as to solve the staining problem of skin, scalp or hair with oil caused by seborrhoea; bactericides for solving the problem of objectionable odor and itching; antiinflammatory agents for solving the problem of skin inflammation; and refrigerants for improving the feel of cosmetics to the touch. However, limitation is placed on the amounts of these medicaments because of their side effects and rather objectionable odor. Thus, satisfactory results are not necessarily obtained.

There is a demand of developing medicated cosmetics which show satisfactory effects of the antiseborrheics, bactericides, antiinflammatory agents and refrigerants used in almost the same amounts as used in prior art.

## SUMMARY OF THE INVENTION

The present inventors made extensive studies on an improvement of the effects of antiseborrheics, bactericides, antiinflammatory agents and refrigerants and, as a result, found that when these medicaments are used in combination with carbon dioxide or substances capable of generating carbon dioxide which is known to have the vasodilative action and is clinically employed as a carbon dioxide bath for rehabilitation, the medicated effects of these agents are drastically improved over the case in which the medicaments are used singly. The present invention is accomplished based on the above finding.

According to the invention, there is provided a medicated cosmetic composition which comprises carbon dioxide or a substance capable of generating carbon dioxide in a cosmetic composition comprising at least one medicament selected from the group consisting of

- 3 -

0170269

antiseborrheics, bactericides, antiinflammatory agents and refrigerants.

### BRIEF DESCRIPTION OF THE DRAWINGS

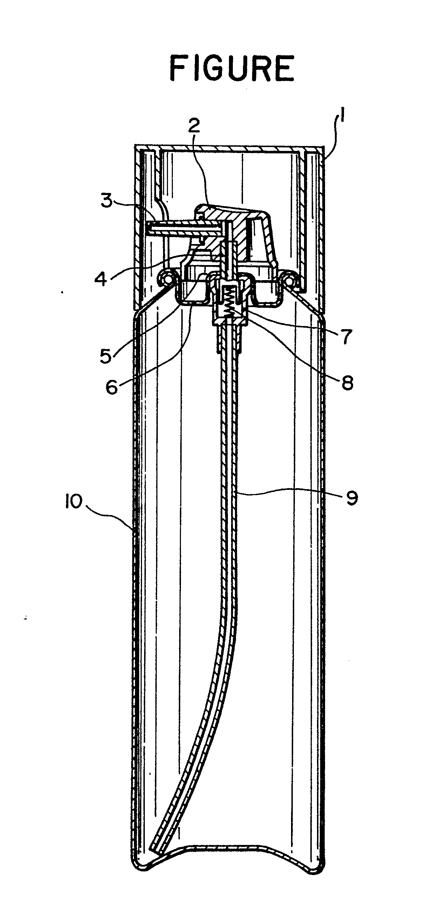

The sole Figure is a schematic sectional view showing one embodiment of a pressure-resistant container having a discharge nozzle which is used to contain medicated cosmetics of the invention.

In the Figure, 1: a cap, 2: a button, 3: a discharge nozzle, 4: a stem, 5: a gasket, 6: a mountain cup, 7: a housing, 8: a spring, 9: a pipe, and 10: a container body.

### DETAILED DESCRIPTION OF THE INVENTION
### AND PREFERRED EMBODIMENTS

The medicated cosmetic compositions of the invention may take various forms.

(1)  A cosmetic composition comprising known medicaments in a pressure-resistant container, into which high pressure carbon dioxide may be blown, or carbon dioxide generator sources such as mixtures of carbonates and acids or dry ice may be charged, followed by sealing the container.

— 4 —

This medicated cosmetic composition is used by discharging the content for application onto a site to be applied.

(2) Carbonates and acids are supported on a carrier such as non-woven fabric, cloth or paper, in a substantially water-free condition. The carrier is further applied with a cosmetic composition comprising at least one medicament.

This medicated cosmetic composition is applied as follows: it is attached to a site to be applied, on which a hot towel may be superposed, or water may be applied, thereby applying water to the cosmetic in order to cause the carbonate and acid to react to generate carbon dioxide.

(3) Carbonates and acids are separately applied to two carriers. Either or both of the carriers may be applied with a cosmetic composition comprising the medicament similar to (2), or may be applied with water.

This type of cosmetic may be superposed on a site to be applied and, if necessary as in the case where the cosmetic is free of water, water is applied similar to (2), thereby generating carbon dioxide.

Of these, the cosmetic (1) is preferred.

The antiseborrheic, which is one of the medicaments of the invention, includes various compounds such as diethylstilbestrol, cycloten acetate, chloromadinon acetate, progesterone, S-carboxy-methyl-L-cystine, oxenedron, androstenedione, testosteron-17-beta-halogenated acetate, deoxycorticosterone and the like. Moreover, there may be further used compounds, known as astringents, such as allantoinchlorohydroxy aluminum, allantoindihydroxy aluminum, aluminum hydroxy chloride, zinc chloride, aluminum chloride, ferric chloride, calamine, dried aluminumpotassium sulfate, zinc paraphenolsulfonate and the like. Of these, diethylstilbestrol and chloromaginon acetate are preferred.

The bactericides include, for example, hinokitiol, salicylic acid, sodium salicylate, paraoxybenzoic acid and esters thereof, isopropylmethylphenol, orthophenylphenol, cresol, thymol, resorcin, photosensitizing dye 101, photosensitizing dye 201, photosensitizing dye 301, photosensitizing dye 401, lysozyme chloride, chlorohexidin hydrochloride, chlorohexidin gluconate, trichlorocarbanilide, hexachlorophene, 2, 2'-dithiobis-pyridine-1, 1'-dioxide and magnesium sulfate trihydrate thereof, zinc-bis(2-pyridylthio)-1, 1'-dioxide, [1-imidazolyl]-[1-(4'-chloro)phenoxy]-3-dimethyl-butane-2-on, monoethanolamine salt of 1-hydroxy-4-methyl-6-(2, 4, 4-trimethylpentyl)-2-(1H)-pyridone and the like. Of these, hinokitiol, thymol, resorcin and trichlorocarbanilide are preferred.

The antiinflammatory agents include, for example, allantoin, ichthammol, guaiazlene, glycylrrhizic acid and salts thereof, glycylrrhetic acid, stearyl glycylrrhetate, glycylrrhetynyl stearate, aloe powder, and the like. Of these, allantoin, dipotassium glycylrrhezate, monoammonium glycylrrhezate, and glycylrrhetic acid are preferred.

The refrigerants are, for example, camphor, menthol, methyl salicylate, cineole, menthone, piperitone, borneol, beta-pinene, menthyl acetate, vanillylamido nonate and the like. Of these, camphor, menthol and piperitone are preferred.

These medicaments are preferably used by dissolving in solvents such as water or water-lower alcohols. Preferably, the medicament is used in amount of from 0.001 to 5 wt% (hereinafter referred to simply as %) of the medicated cosmetic. Less amounts do not lead to satisfactory effects, where larger amounts are

- 7 -

unfavorable because objectionable irritativeness is given to scalp.

Carbon dioxide used in (1) exists as $CO_2$ molecules when a solution dissolving the dioxide is acidic, under which it is known to show the vasodilative action and facilitates the action of percutaneous absorption. The medicated cosmetic composition of the invention has a pH of from 4 to 7, preferably from 4.5 to 6.5, when used in a liquid form. It will be noted that although the pH of the cosmetic becomes more acidic when carbon dioxide is incorporated under pressure and dissolved in the cosmetic composition, the final pH is controlled to be in the above range. The pH modifier used may be organic acids such as citric acid, tartaric acid, lactic acid and the like and salts thereof, phosphoric acid and salts thereof, and solid acids such as acid clay.

The carbonates used in the present invention include, for example, sodium hydrogencarbonate, sodium carbonate, sodium sesqui-carbonate, potassium hydrogencarbonate, potassium carbonate, potassium sesqui-carbonate, ammonium hydrogencarbonate, ammonium carbonate, ammonium sesqui-carbonate, and the like. These carbonates may be used singly or in combination.

The acids may be either organic or inorganic acids, and are preferably water-soluble but solid. Organic acids are, for example, straight-chain fatty acids such as formic acid, acetic acid, propionic acid, butyric acid, valeric acid and the like; dicarboxylic acids such as oxalic acid, maloic acid, succinic acid, glutaric acid, adipic acid, pimelic acid, fumaric acid, maleic acid, phthalic acid, isophthalic acid, terephthalic acid and the like; acidic amino acids such as glutamic acid, aspartic acid and the like; oxyacids such as glycolic acid, lactic acid, hydroxyacrylic acid, alpha-oxybutyric acid, glyceric acid, tartoronic acid, malic acid, tartaric acid, citric acid, salicylic acid (o, m and p), gallic acid, mandelic acid, tropic acid, ascorbic acid, gluconic acid and the like; cinnamic acid, benzoic acid, phenylacetic acid, nicotinic acid, kainic acid, sorbic acid, pyrrolidonecarboxylic acid, trimellitic acid, benzenesulfonic acid, toluenesulfonic acid; and acid salts thereof. Examples of the inorganic acids include phosphoric acid, potassium dihydrogenphosphoric acid, sodium dihydrogenphosphoric acid, sodium sulfite, potassium sulfite, sodium pyrosulfite (sodium metabisulfite), potassium pyrosulfite (potassium metabisulfite), acidic sodium hexametaphosphate, acidic potassium hexametaphosphate, acidic sodium pyrophosphate, acidic pottasium pyrophosphate, sulfamine and the like. Of these, aliphatic dicarboxylic acids such as succinic acid and the like, fumaric acid, phosphoric acid, and acidic salts thereof are preferred.

In the practice of the invention, the amounts of these carbonates and acids are preferably so controlled that the pH of the carbon dioxide-generating medium is in the range of from 4 to 7. For instance, when used in combination with a cosmetic composition comprising the medicaments, carbonates and acids are, respectively, used in an amount of from 1 to 20%, preferably from 2 to 10%, based on the total composition. The concentration of carbon dioxide in the medicated cosmetic composition of the invention is preferably in the range not less than 60 ppm. Less concentrations do not lead to satisfactory results.

The medicated cosmetic composition in the form of (1) is preferably charged in a pressure-resistant container having a discharge nozzle. For the preparation of the medicated cosmetic composition of (1), various methods are used including a method in which a cosmetic composition is charged into a pressure-resistant container, then a high pressure gas is charged and the container is sealed, a method in which a cosmetic composition comprising a carbonate such as sodium hydrogencarbonate is charged into a pressure-resistant container, to which a pH modifier is added so as to generate carbon dioxide, and then the container is sealed, and a method in which dry ice pellets are placed in a container which is subsequently sealed. Of these, the method using the high pressure gas in preferred.

In the above methods, part of carbon dioxide is dissolved in cosmetic and part of the carbon dioxide is present as a gas in the container. In the present invention, it is important that carbon dioxide exists in cosmetic compositions as being partially dissolved. The amount of carbon dioxide in the composition should preferably be not less than 60 ppm. Less amounts are not favorable since the effects of the medicated agents cannot be expected. The amount of carbon dioxide may be controlled by controlling a carbon dioxide charge. It is preferable that carbon dioxide is charged so that the pressure in the container reaches 1.2 to 8 $kg/cm^2$ (gage pressure) at a temperature of 35°C.

The pressure-resistant container used in the present invention should withstand the pressure to keep the cosmetic composition in a sealed condition after preparation. For instance, there are used metallic containers such as aluminum or tin, synthetic resin containers such as acetal resins and polycarbonate resins, and glass containers.

When the discharge nozzle has a small diameter, the content is sprayed as mist, whereas when the diameter is large, the content is sprayed as foams or droplets. The diameter of the nozzle may vary depending on the inner pressure in the container. In general, the diameter of a discharge nozzle of an aerosol sprayer is 0.3 mm or below. when this ordinary type of nozzle is used for the cosmetic composition of the invention, the content is sprayed in the form of mist, during which carbon dioxide is rapidly flown out, so that the purpose of the invention cannot be achieved. In order to enhance the effects of the invention, it is necessary to keep carbon dioxide in the cosmetic composition over a long time. In addition, the cosmetic composition of the invention should preferably be applied directly on scalp, without attachment to hair, in the form of foams or liquid. On the scalp, the composition is allowed to foam. To this end, the discharge nozzle should preferably have a diameter of from 0.3 to 1.5 mm and a length of 1 to 15 cm. In order to prevent reduction of dissolved carbon dioxide accompanied by reduction of the inner pressure in the container after use so that the spraying condition is held constant, a mini bomb of carbon dioxide may be built in the container. When the pressure is reduced on use, fresh carbon dioxide is supplied from the mini bomb. This type of aerosol injector is proposed in Japanese Laid-open Patent Application No. 57-153752. In doing so, it becomes possible to keep a concentration of carbon dioxide at a constant level from an initial stage till completion of use.

The medicated cosmetic compositions of the invention may take various forms including, for example, lotion, emulsion, hair lotion, hair tonic, hair liquid, shampoo, rinse, hair grower and the like. Aside from the essential ingredients, ingredients which are used in ordinary cosmetics may be added to the cosmetic composition of the invention. Examples of such ingredients include oil bases, emollients, gelling agents, various emulsifiers, perfumes, preservatives such as parahydroxybenzoates, antioxidants such as butylhydroxyanisole, colorants such as dyes, humectants such as propylene glycol, film-forming agents,

0170269

thickeners, blood circulation accelerators, vitamins and the like.

The cosmetic composition of the invention is preferably used as a hair tonic using a water/ethanol base.

[Effects]

The medicated cosmetics of the invention obtained as described above can remarkably improve the medical efficacies such as the antiseborrheic action, antiinflammatory action and refrigerating feel even though the medicaments are used almost in the same amounts as in prior art.

The present invention is more particularly described by way of examples.

Example 1

Hair Tonic (having Antiseborrheic Action):

Hair tonics having the compositions indicated in Table 1 were prepared and evaluated with respect to oiliness and stickiness of hair when applied to hair. The evaluation was organoleptically effected by a method in which after washing of hair, hair tonics according to invention and comparative hair tonics were applied every day, followed by inhibiting washing of the applied hair

- 14 -

over 3 days and subjecting such hair to organoleptic
test by ten expert panelers.  The absolute evaluation
was effected according to the following criteria.

Oiliness of Hair: (+2) no oiliness, (+1)
fairly free of oiliness, (0) moderate, (-1) slightly
oily, (-2) oily

Stickiness of hair: (+2) no stickiness, (+1)
fairly free of stickiness, (0) moderate, (-1) slightly
sticky, (-2) sticky

The results are shown in Table 2.

(Composition)

Table 1

| | Inventive Products | | Comparative Products | | | |
| --- | --- | --- | --- | --- | --- | --- |
| | (A) | (B) | (a) | (b) | (c) | (d) |
| Carbon dioxide | 1.00 % | 1.00 % | – % | – % | – % | – % |
| **Antiseborrheics** | | | | | | |
| Diethylstylbestrol | 0.002 | – | 0.002 | – | – | – |
| Chloromadinon Acetate | – | 0.5 | – | 0.5 | 1.0 | 2.0 |
| **pH Modifiers** | | | | | | |
| Lactic Acid | 0.133 | 0.133 | 0.133 | 0.133 | 0.133 | 0.133 |
| Sodium Lactate | 0.3 | 0.3 | 0.3 | 0.3 | 0.3 | 0.3 |
| 50V/V % Aqueous Ethanol | balance | balance | balance | balance | balance | balance |

(Preparation)

An antiseborrheic and a pH modifier were added to a 50 v/v% aqueous ethanol solution and well agitated, followed by charging into a pressure-resistant container as shown in the Figure, injecting carbon dioxide into the container and sealing.

(Results)

Table 2

|  | | Oiliness of Hair | Stickiness of Hair |
|---|---|---|---|
| Inventive Product | (A) | 1.2 | 1.0 |
| " | (B) | 1.6 | 1.3 |
| Comparative Product | (a) | -1.0 | -1.2 |
| " | (b) | -0.6 | -1.0 |
| " | (c) | -0.2 | -0.6 |
| " | (d) | -0.2 | -0.2 |

Each value in the table is an average of values evaluated by ten panelers.

As will be seen from the above results, the hair tonics according to the invention in which carbon dioxide and an antiseborrheic are used are better in non-oiliness and stickiness than the comparative hair tonics.

Example 2

Hair Tonic (having the Bactericidal Action):

Hair tonics having the compositions indicated in Table 3 were prepared and evaluated with respect to the effect of preventing objectionable odor from and itching of scalp. The evaluation was effected as follows: 10 persons whose hair had been washed made use of the respective hair tonics according to the invention and for comparison for 3 days without washing the hair; and then the odor of scalp was organoleptically evaluated by expert panelers and the itching was assessed by the tested persons. The absolute evaluation was effected according to the following criteria.

Odor of scalp: (+2) no odor, (+1) substantially free of odor, (0) moderate, (-1) slight offensive odor, (-2) fair offensive odor

Itching of scalp: (+2) no itching, (+1) substantially free of itching, (0) moderate, (-1) slight itching, (-2) fair itching

The results are shown in Table 4.

Table 3

| | Inventive Products | | | Comparative Products | | | | |
|---|---|---|---|---|---|---|---|---|
| | (C) | (D) | (E) | (e) | (f) | (g) | (h) | (i) |
| Carbon Dioxide | 1.00 % | 1.00 % | 1.00 % | – % | – % | – % | – % | – % |
| **Bactericides** | | | | | | | | |
| Thymol | 0.1 | – | – | 0.1 | – | – | – | – |
| Resorcin | – | 0.1 | – | – | 0.1 | – | – | – |
| Trichloro Carbanilide | – | – | 0.1 | – | – | – | 0.2 | 0.3 |
| **pH Modifiers** | | | | | | | | |
| Lactic Acid | 0.133 | 0.133 | 0.133 | 0.133 | 0.133 | 0.133 | 0.133 | 0.133 |
| Sodium Lactate | 0.3 | 0.3 | 0.3 | 0.3 | 0.3 | 0.3 | 0.3 | 0.3 |
| 50 V/V% Aqueous Ethanol | bal-ance | bal-ance | bal-ance | bal-ance | bal-ance | bal-ance | bal-ance | bal-ance |

(Preparation)

A bactericide and a pH modifier were added to a 50 v/v% aqueous ethanol solution and well agitated, followed by charging into a pressure-resistant container which is the same type as shown in Figure, injecting carbon dioxide into the container and sealing.

- 19 -

(Results)

Table 4

|  |  | Scalp Odor | Itchy Feel in the Scalp |
|---|---|---|---|
| Inventive Product | (C) | 1.6 | 1.2 |
| " | (D) | 1.6 | 1.4 |
| " | (E) | 1.4 | 1.2 |
| Comparative Product | (e) | 0.0 | -0.8 |
| " | (f) | -0.8 | -0.6 |
| " | (g) | -0.8 | -0.6 |
| " | (h) | -0.4 | -0.2 |
| " | (i) | 0.0 | -0.1 |

Each value in the table is an average of values evaluated by ten panelers.

From the results of the above tables, it will be clear that the hair tonics according to the invention in which carbon dioxide and bactericides are used are better than the comparative hair tonics with respect to odor and itching of scalp.

**Example 3**

Hair Tonic (having Antiinflammatory Action):

Hair tonics having compositions indicated in Table 5 were prepared and used for evaluation of the antiphlogistic action and the texture of hair. The evaluation was effected by applying each hair tonic to 10 panelers for 5 days, during which washing of the hair was inhibited. The panelers complaining of itching of scalp were organoleptically examined. The absolute evaluation was made according to the following criteria.

Antiinflammatory action: (+3) no itching, (+2) substantially free of itching, (+1) a reduced degree of itching, (0) no change, Texture of hair: (+1) good, (0) no difference, (-1) bad The results are shown in Table 6.

Table 5

| | Inventive Products | | | Comparative Products | | | | |
| | (F) | (G) | (H) | (j) | (k) | (1) | (m) | (n) |
|---|---|---|---|---|---|---|---|---|
| | % | % | % | % | % | % | % | % |
| Carbon Dioxide | 1.00 | 1.00 | 1.00 | – | – | – | – | – |
| **Antiinflammatory agents** | | | | | | | | |
| Allantoin | 0.1 | – | – | 0.1 | – | – | – | – |
| Dipotassium Glycyrrhizinate | – | 0.1 | – | – | 0.1 | – | 0.2 | 0.3 |
| Monoammonium Glycyrrhizinate | – | – | 0.1 | – | – | 0.1 | – | – |
| **pH Modifiers** | | | | | | | | |
| Lactic Acid | 0.133 | 0.133 | 0.133 | 0.133 | 0.133 | 0.133 | 0.133 | 0.133 |
| Sodium Lactate | 0.3 | 0.3 | 0.3 | 0.3 | 0.3 | 0.3 | 0.3 | 0.3 |
| 50 V/V% Aqueous Ethanol | balance | balance | balance | balance | balance | balance | balance | balance |

(Preparation)

An antiinflammatory agent and a pH modifier were added to a 50 v/v% aqueous ethanol solution and well agitated, followed by charging into a pressure-resistant container which is the same type as shown in the Figure, injecting carbon dioxide into the container and sealing.

(Results)

Table 6

|  |  | of | Antiinflammatory Action | Texture Hair |
| --- | --- | --- | --- | --- |
| Inventive Product | (F) | | 2.5 | 0.1 |
| " | (G) | | 2.6 | 0.0 |
| " | (H) | | 2.8 | 0.1 |
| Comparative Product | (i) | | 1.8 | 0.0 |
| " | (k) | | 1.5 | 0.1 |
| " | (l) | | 1.6 | 0.0 |
| " | (m) | | 2.0 | -0.2 |
| " | (n) | | 2.2 | -0.5 |

Each value in the table is an average of values evaluated by ten panelers.

From the above results, it will be clear that the hair tonics of the invention in which carbon dioxide and antiinflammatory agents are used, are better than the comparative tonics with respect to the antiinflammatory action without impeding the feel of hair.

Example 4

- 23 -

Hair Tonics (having the Refrigerating Feel) Hair tonics having the compositions indicated in Table 7 were prepared and evaluated by ten panelers with respect to the feel of refrigeration and odor. The evaluation was shown in terms of absolute evaluation according to the following criteria. The results are shown in Table 8.

Table 7

| | Inventive Products | | | Comparative Products | | | | |
|---|---|---|---|---|---|---|---|---|
| | (I) | (J) | (K) | (o) | (p) | (q) | (r) | (s) |
| Carbon Dioxide | 1.00 (%) | 1.00 (%) | 1.00 (%) | – | – | – | – | – |
| **Refrigerants** | | | | | | | | |
| Menthol | 0.05 | – | – | 0.05 (%) | – | – | 0.1 (%) | 0.2 (%) |
| Camphor | – | 0.1 | – | – | 0.1 (%) | – | – | – |
| Piperitone | – | – | 0.1 | – | – | 0.1 | – | – |
| **pH Modifiers** | | | | | | | | |
| Lactic Acid | 0.133 | 0.133 | 0.133 | 0.133 | 0.133 | 0.133 | 0.133 | 0.133 |
| Sodium Lactate | 0.3 | 0.3 | 0.3 | 0.3 | 0.3 | 0.3 | 0.3 | 0.3 |
| 50 V/V% Aqueous Ethanol | balance | balance | balance | balance | balance | balance | balance | balance |

(Preparation)

A refrigerant and a pH modifier were added to a 50 v/v% aqueous ethanol solution and well agitated, followed by charging into a pressure-resistant container which is the same type as shown in the Figure, injecting carbon dioxide into the container and sealing.

(Evaluation Criteria) +3   very intense +2   intense +1   slight

0   no

(Results)

Table 8

|  |  | Refrigerating Feel | Odor |
|---|---|---|---|
| Inventive Product | (I) | 2.8 | 0.2 |
| " | ( ) | 2.5 | 0.2 |
| " | (K) | 2.7 | 0.3 |
| Comparative Product | (o) | 1.2 | 0.2 |
| " | (p) | 1.0 | 0.1 |
| " | (q) | 1.5 | 0.3 |
| " | (r) | 1.8 | 1.1 |
| " | (s) | 2.5 | 2.6 |

Each value in the table is an average of values evaluated by ten panelers.

From the above results, it will be clear that the hair tonics of the invention in which carbon dioxide and refrigerants are used, are better than the comparative tonics with respect to the feel of refrigeration and less in odor.

Example 5

Hair Tonic (having Antiseborrheic and Bactericidal Action):

(Composition)

| | | | |
|---|---|---|---|
| (1) | Ethanol | | 50.0 (%) |
| (2) | Carbon dioxide | | 1.0 |
| (3) | Chloromadinon acetate (antiseborrheic) | | 0.5 |
| (4) | Hinokitiol (bactericide) | | 0.05 |
| (5) | Lactic acid | | 0.133 |
| (6) | Sodium lactate | | 0.3 |
| (7) | Perfume | | suitable amount |
| (8) | Water | | balance |

(Preparation)

(1), (3) to (8) were mixed at room temperature and charged into a pressure-resistant container which is the same type as shown in the Figure, to which an injector was attached for sealing. (2) was then charged to obtain a final product. The resultant hair tonic had good antiseborrheic and bactericidal actions, imparting good feel of the hair and permitting scalp to be free of itching.

Example 6

Hair Tonic (having Bactericidal Action):

(Composition)

| | | | |
|---|---|---|---|
| (1) | Ethanol | | 50.0 (%) |

| | | |
|---|---|---|
| (2) | Carbon dioxide | 1.0 |
| (3) | Resorcin | 0.1 |
| (4) | Polyoxyethylene hardened castor oil (20 E.O.) | 0.5 |
| (5) | Lactic acid | 0.133 |
| (6) | Sodium lactate | 0.3 |
| (7) | Perfume | suitable amount |
| (8) | Water | balance |

(Preparation)

(1), (3) to (8) were mixed at room temperature and charged into a pressure-resistant container which is the same type as shown in the Figure, to which an injector was attached for sealing. (2) was then charged to obtain a final product. The resultant hair tonic had good effects of preventing offensive odor and itching of scalp.

Example 7

Hair Tonic (having Antiinflammatory and Bactericidal Action):

(Composition)

| | | |
|---|---|---|
| (1) | Ethanol | 50.0 (%) |
| (2) | Carbon dioxide | 1.0 |

| | | |
|---|---|---|
| (3) | Dipotassium glycylrrhizate (antiinflammatory agent) | 0.1 |
| (4) | Hinokitiol (bactericide) | 0.05 |
| (5) | Lactic acid | 0.133 |
| (6) | Sodium lactate | 0.3 |
| (7) | Perfume | suitable amount |
| (8) | Water | balance |

(Preparation)

(1), (3) to (8) were mixed at room temperature and charged into a pressure-resistant container which is the same type as shown in the Figure, to which an injector was attached for sealing. (2) was then charged to obtain a final product. The resultant hair tonic had good antiinflammatory and bactericidal actions, without impeding the good feel of hair.

Example 8

Hair Tonic (having Bactericidal Action and Refrigerating Feel):

(Composition)

| | | |
|---|---|---|
| (1) | Ethanol | 50.0 (%) |
| (2) | Carbon dioxide | 1.0 |
| (3) | Menthol (refrigerant) | 0.05 |

| | | |
|---|---|---|
| (4) | Hinokitiol (bactericide) | 0.05 |
| (5) | Lactic acid | 0.133 |
| (6) | Sodium lactate | 0.3 |
| (7) | Perfume | suitable amount |
| (8) | Water | balance |

(Preparation)

(1), (3) to (8) were mixed at room temperature and charged into a pressure-resistant container which is the same type as shown in the Figure, to which an injector was attached for sealing. (2) was then charged to obtain a final product. The resultant hair tonic had good bactericidal action and good feel of refrigeration, without involving the odor of menthol.

Example 9

Skin Lotion (having Antiseborrheic Action):

(Composition)

| | | |
|---|---|---|
| (1) | Ethanol | 10.0 (%) |
| (2) | Carbon dioxide | 1.0 |
| (3) | Diethylstilbestrol | 0.002 |
| (4) | dl-alpha-Tocopheryl acetate | 0.05 |
| (5) | Lactic acid | 0.133 |
| (6) | Sodium lactate | 0.3 |

(7)  Polyoxyethylene hardened castor oil
     (20 E.O.)                                    0.5

(8)  Perfume                            suitable amount

(9)  Water                                     balance

(Preparation)

(1), (3) to (9) were mixed at room temperature and charged into a pressure-resistant container which is the same type as shown in the Figure, to which an injector was attached for sealing.  (2) was then charged to obtain a final product.  The resultant skin lotion had good antiseborrheic action and the skin was not stained oilly without being dry of skin.

Example 10

Skin Lotion (having Antiseborrheic Action):

The procedure of Example 9 was repeated except that diethylstilbestrol (3) was replaced by 0.5% of aluminum chloride, thereby obtain a skin lotion.

Example 11

Skin Lotion (having Bactericidal Action):
(Composition)

(1)  Ethanol                                 10.0 (%)

(2)  Carbon dioxide                            1.0

- 31 -

| (3) | Trichlorocarbanilide | 0.1 |
|-----|----------------------|-----|
| (4) | dl-alpha-Tocopheryl acetate | 0.05 |
| (5) | Lactic acid | 0.133 |
| (6) | Sodium lactate | 0.3 |
| (7) | Polyoxyethylene hardened castor oil (20 E.O.) | 0.5 |
| (8) | Perfume | suitable amount |
| (9) | Water | balance |

(Preparation)

(1), (3) to (9) were mixed at room temperature and charged into a pressure-resistant container which is the same type as shown in the Figure, to which an injector was attached for sealing. (2) was then charged to obtain a final product. The resultant skin lotion had a good effect of preventing the offensive odor of the armpit and sweat.

Example 12

Skin Lotion (having Antiinflammatory Action):

(Composition)

| (1) | Ethanol | 10.0 (%) |
|-----|---------|----------|
| (2) | Carbon dioxide | 1.0 |
| (3) | Allantoin | 0.1 |

| (4) | dl-alpha-Tocopheryl acetate | 0.05 |
|-----|------------------------------|------|
| (5) | Lactic acid | 0.133 |
| (6) | Polyoxyethylene hardened castor oil (20 E.O.) | 0.5 |
| (7) | sodium lactate | 0.3 |
| (8) | Perfume | suitable amount |
| (9) | Water | balance |

(Preparation)

(1), (3) to (9) were mixed at room temperature and charged into a pressure-resistant container which is the same type as shown in the Figure, to which an injector was attached for sealing. (2) was then charged to obtain a final product. The resultant skin lotion had a good antiinflammatory action.

Example 13

Skin Lotion (having Refrigerating Feel):

(Composition)

| (1) | Ethanol | 10.0 (%) |
|-----|---------|----------|
| (2) | Carbon dioxide | 1.0 |
| (3) | Camphor | 0.1 |
| (4) | dl-alpha-Tocopheryl acetate | 0.05 |
| (5) | Lactic acid | 0.133 |

| | | |
|---|---|---|
| (6) | Sodium lactate | 0.3 |
| (7) | Polyoxyethylene hardened castor oil (20 E.O.) | 0.5 |
| (8) | Perfume | suitable amount |
| (9) | Water | balance |

(Preparation)

(1), (3) to (9) were mixed at room temperature and charged into a pressure-resistant container which is the same type as shown in the Figure, to which an injector was attached for sealing. (2) was then charged to obtain a final product. The resultant skin lotion had a good feel of refrigeration without involving any camphor smell.

Example 14

Shampoo (having Antiseborrheic Action):

(Composition)

| | | |
|---|---|---|
| (1) | Polyoxyethylene(2)sodium lauryl sulfate | 15.0 (%) |
| (2) | Coconut fatty acid diethanolamide | 3.0 |
| (3) | Carbon dioxide | 1.0 |
| (4) | Chloromadinon acetate | 0.5 |
| (5) | Lactic acid | 0.133 |
| (6) | Sodium lactate | 0.3 |

| (7) | Perfume | suitable amount |
|-----|---------|-----------------|
| (8) | Water | balance |

(Preparation)

(1), (2), (4) to (8) were mixed at room temperature and charged into a pressure-resistant container which is the same type as shown in the Figure, to which an injector was attached for sealing. (3) was then charged to obtain a final product. The resultant shampoo had a good antiseborrheic action.

Example 15

· Shampoo (having Bactericidal Action):

(Composition)

| (1) | Polyoxyethylene(2)sodium lauryl sulfate | 15.0 (%) |
|-----|------------------------------------------|----------|
| (2) | Coconut fatty acid diethanolamide | 3.0 |
| (3) | Carbon dioxide | 1.0 |
| (4) | Thymol | 0.1 |
| (5) | Lactic acid | 0.133 |
| (6) | Sodium lactate | 0.3 |
| (7) | Perfume | suitable amount |
| (8) | Water | balance |

(Preparation)

(1), (2), (4) to (8) were mixed at room temperature and charged into a pressure-resistant container which is the same type as shown in the Figure, to which an injector was attached for sealing. (3) was then charged to obtain a final product. The resultant shampoo had a good effect of preventing the smell and itching of scalp.

Example 16

Shampoo (having Antiinflammatory Action):

(Composition)

|     |     |     |
|-----|-----|-----|
| (1) | Polyoxyethylene(2)sodium lauryl sulfate | 15.0 (%) |
| (2) | Coconut fatty acid diethanolamide | 3.0 |
| (3) | Carbon dioxide | 1.0 |
| (4) | Glycylrrhizin | 0.05 |
| (5) | Lactic acid | 0.133 |
| (6) | Sodium lactate | 0.3 |
| (7) | Perfume | suitable amount |
| (8) | Water | balance |

(Preparation)

(1), (2), (4) to (8) were mixed at room temperature and charged into a pressure-resistant container which is the same type as shown in the Figure, to which an injector was attached for sealing. (3) was then charged to obtain a final product. The resultant shampoo had a good antiinflammatory action.

Example 17

Shampoo (having Refrigerating Feel):

(Composition)

| | | |
|---|---|---|
| (1) | Polyoxyethylene(2)sodium lauryl sulfate | 15.0 (%) |
| (2) | Coconut fatty acid diethanolamide | 3.0 |
| (3) | Carbon dioxide | 1.0 |
| (4) | Piperitone | 0.5 |
| (5) | Lactic acid | 0.133 |
| (6) | Sodium lactate | 0.3 |
| (7) | Perfume | suitable amount |
| (8) | Water | balance |

(Preparation)

(1), (2), (4) to (8) were mixed at room temperature and charged into a pressure-resistant container which is the same type as shown in the Figure, to which an injector was attached for sealing. (3) was then charged to obtain a final product. The resultant shampoo had a good feel of refrigeration.


**Example 18**

Hair Rinse (having Antiseborrheic Action):

(Composition)

| | | |
|---|---|---|
| (1) | Distearyldimethylammonium chloride | 2.0 (%) |
| (2) | Stearyl alcohol | 1.0 |
| (3) | Carbon dioxide | 1.0 |
| (4) | Diethylstilbestrol | 0.002 |
| (5) | Lactic acid | 0.133 |
| (6) | Sodium lactate | 0.3 |
| (7) | Perfume | suitable amount |
| (8) | Water | balance |


(Preparation)

(1), (2), (4) and (7) were heated and mixed at 70°C, to which a mixture of (5), (6) and (8) which had been mixed, melted and heated to 70°C, was added and emulsified. The mixture was cooled down to room temperature and placed in a pressure-resistant container which is the same type as shown in the Figure, which was then attached with an injector and sealed. (3) was charged into the container to obtain a final product. The resultant hair rinse had an excellent antiseborrheic action.

Example 19

Hair Rinse (having Bactericidal Action):

(Composition)

| | | |
|---|---|---|
| (1) | Distearyldimethylammonium chloride | 2.0 (%) |
| (2) | Stearyl alcohol | 1.0 |
| (3) | Carbon dioxide | 1.0 |
| (4) | Resorcin | 0.1 |
| (5) | Lactic acid | 0.133 |
| (6) | Sodium lactate | 0.3 |
| (7) | Perfume | suitable amount |
| (8) | Water | balance |

(Preparation)

(1), (2), (4) and (7) were heated and mixed at 70°C, to which a mixture of (5), (6) and (8) which had been mixed, melted and heated to 70°C, was added and emulsified. The mixture was cooled down to room temperature and placed in a pressure-resistant container which is the same type as shown in the Figure, which was then attached with an injector and sealed. (3) was charged into the container to obtain a final product.

The resultant hair rinse had a good effect of preventing the smell and itching of scalp.

**Example 20**

Hair Rinse (having Antiinflammatory Action):
(Composition)

|     |                                      |                 |
| --- | ------------------------------------ | --------------- |
| (1) | Distearyldimethylammonium chloride   | 2.0 (%)         |
| (2) | Stearyl alcohol                      | 1.0             |
| (3) | Carbon dioxide                       | 1.0             |
| (4) | Allantion                            | 0.1             |
| (5) | Lactic acid                          | 0.133           |
| (6) | Sodium lactate                       | 0.3             |
| (7) | Perfume                              | suitable amount |
| (8) | Water                                | balance         |

(Preparation)

(1), (2), (4) and (7) were heated and mixed at 70°C, to which a mixture of (5), (6) and (8) which had been mixed, melted and heated to 70°C, was added and emulsified. The mixture was cooled down to room temperature and placed in a pressure-resistant container which is the same type as shown in the Figure, which was then attached with an injector and sealed. (3) was charged into the container to obtain a final product.

The resultant hair rinse had a good antiinflammatory action.

Example 21

Hair Rinse (having Refrigerating Feel):

(Composition)

| | | |
|---|---|---|
| (1) | Distearyldimethylammonium chloride | 2.0 (%) |
| (2) | Stearyl alcohol | 1.0 |
| (3) | Carbon dioxide | 1.0 |
| (4) | Menthol | 0.2 |
| (5) | Lactic acid | 0.133 |
| (6) | Sodium lactate | 0.3 |
| (7) | Perfume | suitable amount |
| (8) | Water | balance |

(Preparation)

(1), (2), (4) and (7) were heated and mixed at 70°C, to which a mixture of (5), (6) and (8) which had been mixed, melted and heated to 70°C, was added and emulsified. The mixture was cooled down to room temperature and placed in a pressure-resistant container which is the same type as shown in the Figure, which was then attached with an injector and sealed. (3) was charged into the container to obtain a final product. The resultant hair rinse had a good feel of refrigeration.

WHAT IS CLAIMED IS:

1. A medicated cosmetic composition characterized by incorporating carbon dioxide or a substance capable of emitting carbon dioxide in a cosmetic composition comprising at least one medicament selected from the group consisting of antiseborrheics, bactericides, antiinflammatory agents and refrigerants.

2. A medicated cosmetic composition according to Claim 1, wherein said at least one medicament is added in an amount of from 0.001 to 5 wt%.

3. A medicated cosmetic composition according to Claim 1, wherein the amount of carbon dioxide is not less than 60 ppm.

4. A medicated cosmetic composition according to Claim 1, wherein said composition is charged in a pressure-resistant container having a discharge nozzle.

5. A medicated cosmetic composition according to Claim 1, wherein said discharge nozzle has a diameter of from 0.3 to 1.5 mm and said composition is charged such that the pressure in the pressure-resistant container ranges from 1.2 to 8 $kg/cm^2$ (gage pressure) at 35°C.

0170269

# FIGURE